# EUROPÄISCHE PATENTANMELDUNG

(11) **EP 0 733 372 A2**
(43) Veröffentlichungstag der Anmeldung: **25.09.1996**
(21) Anmeldenummer: 96810151.9
(22) Anmeldetag: 12.03.1996
(51) Int. Cl.: A61K 47/00, A61K 9/10

(54) **Pharmazeutische Formulierungsgrundlage für Nanosuspensionen**

(30) Priorität: 21.03.1995 CH 804/95
(71) Anmelder: CIBA-GEIGY AG, 4002 Basel (CH); VESIFACT AG, CH-6340 Baar (CH)
(72) Erfinder: Weder, Hans Georg, Prof.Dr., 8803 Rüschlikon (CH); van Hoogevest, Peter, Dr., 4125 Riehen (CH)

(57) **Zusammenfassung**

Die vorliegende Erfindung betrifft eine Formulierungsgrundlage zur Herstellung einer pharmazeutischen Zusammensetzung für die intravenöse Applikation des schwerlöslichen Staurosporin-Derivats N-Benzoylstaurosporin. Die Zusammensetzung enthält folgende bevorzugte Komponenten:
a) den Wirkstoff N-Benzoyl-Staurosporin;
b) ein Polyoxyethylen-polyoxypropylen-Blockcopolymer;
c) Ethanol und Wasser als Trägerflüssigkeiten; und
d) gereinigtes Lecithin aus Sojabohnen und
e) als wasserlösliche Hilfsstoffe Glycerin und Sorbitol.

## Beschreibung

Die vorliegende Erfindung betrifft eine Formulierungsgrundlage für die Herstellung einer pharmazeutischen Zusammensetzung für die intravenöse Applikation eines schwerlöslichen Staurosporin-Derivats, Verfahren zur Herstellung dieser Formulierungsgrundlage und ihre Weiterverarbeitung zu einem pharmazeutischen Präparat.

Das Ausgangsmaterial zahlreicher Derivate, Staurosporin, wurde 1977 aus den Kulturen von Streptomyces staurosporeus AWAYA, TAKAHASHI, OMURA SP. NOV. AM 2282, vgl. S.Omura et al., J. Ant. 30, 275-281 (1977), isoliert. Für das Grundgerüst wurde zuerst die relative und danach die absolute Konfiguration ermittelt, siehe N. Fumato et al., Tetrahedron Letters 35: 8, 1251-1254 (1994). Dem besonders bevorzugten N-Benzoyl-staurosporin-Derivat, welches in der U.S. Patentschrift 5 093 330 beschrieben ist, lässt sich folgende Strukturformel zuordnen: 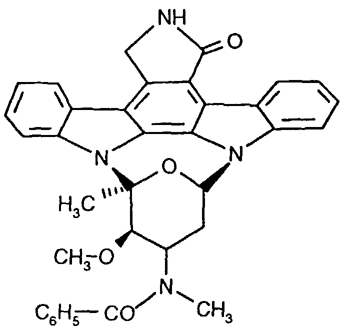 Staurosporin sowie seine Derivate, wie N-Benzoyl-Staurosporin, weisen eine starke Hemmung von Proteinkinase C auf. Sie hemmen aber auch ebenso andere Proteinkinasen. Sie eignen sich therapeutisch für verschiedene Indikationen, insbesondere als Tumorhemmer, als Antiinflammatorika, als Antibiotika, bei der Behandlung von Arteriosklerose und diversen Erkrankungen des kardiovaskulären Systems und des zentralen Nervensystems. Für Staurosporin und die meisten Derivate ist die äusserst geringe Wasserlöslichkeit charakteristisch, was ihre Verwendbarkeit für intravenöse Darreichungsformen bisher ausserordentlich erschwert.

Obwohl perorale Darreichungsformen, wie Tabletten oder Kapseln, zunehmende Bedeutung erlangen, bleiben intravenöse Darreichungsformen trozt gewisser Nachteile weiterhin relevant. Den Nachteilen, z.B. Vornahme der Verabreichung nur durch den Arzt bzw. besonders beauftragtem Hilfspersonal und Geschicklichkeitsanforderungen an die verabreichende Person, "psychologischen" Problemen beim Patienten und seinem Schmerzempfinden und hohem technischen Aufwand bei der Herstellung dieser Darreichungsformen, stehen eindeutige Vorteile gegenüber. Bei direkter intravenöser Applikation eines Wirkstoffs lässt sich der Metabolismus im Gastrointestinaltrakt weitgehend umgehen, dem oral applizierte Wirkstoffe stets unterworfen sind. So wird insbesondere der sogenannte "First-Pass-Effekt" durch die Leberpassage minimiert. Manche Wirkstoffe lassen sich ausschliesslich nur intravenös applizieren, die oral ungenügend resorptionsfähig wären. Andere Wirkstoffe kann man intravenös in einer geringeren Dosis wirksam applizieren als für die orale Applikation erforderlich wäre. Generell steht bei lebensbedrohenden Krankheiten, wie Tumorerkrankungen, die intravenöse Applikation im Vordergrund, da man die Problematik der Resorption durch den Gastrointestinaltrakt verbunden mit unerwünschtem Metabolismus nicht hinnehmen kann.

Für die wichtige Wirkstoffgruppe der Staurosporine bzw. Derivate des Staurosporins hat bisher noch keine geeignete intravenöse Darreichungsform zur Verfügung gestanden. Der vorliegenden Erfindung liegt die Aufgabe zugrunde, für Staurosporinderivate, insbesondere für N-Benzoyl-staurosporin, eine geeignete intravenöse Darreichungsform zur Verfügung zu stellen.

Zahlreiche Publikationen schlagen verschiedene Möglichkeiten vor, einen schwerlöslichen Wirkstoff in eine für intravenöse Formulierungen geeignete, löslichere Form zu überführen. Dies kann beispielsweise durch Zusatz von Solubilisatoren, wie 1,2-Propylenglycol oder Polyethylenglycol 300 - 400, geschehen. Bei mangelnder Löslichkeit trotz Verwendung der wenigen in den nationalen Pharmakopöen und Arzneibüchern zugelassenen Solubilisatoren bieten sich gemäss Stand der Technik feindisperse Systeme auf der Basis von Lipidgemischen an. Darin ist der schwerlösliche Wirkstoff in Lipidpartikeln mit einer Teilchengrösse kleiner als 1 µm eingeschlossen und bildet mit der wässrigen Trägerflüssigkeit ein kolloiddisperses oder vorzugsweise feindisperses System, das zwar keine echte molekular disperse Lösung darstellt, aber für eine intravenöse Darreichungsform immer noch ausreichend homogen ist. Zahlreiche Publikationen schlagen die Verkapselung von schwerlöslichen Wirkstoffen in Mizellen, Mischmizellen, Umkehrmizellen oder unilamellaren oder multilamellaren Liposomen vor.

Diese Verfahren weisen den eindeutigen Vorteil auf, dass man mit ihnen auch noch Wirkstoffe mit ausgeprägter Schwerlöslichkeit in intravenöse Darreichungsformen überführen kann. Nachteilig sind aber häufig auftretende, bekannte Probleme wie ungenügende Stabilität der Dispersion, zu geringe Mengen an verkapseltem Wirkstoff, starke Abhängigkeit der Partikelgrösse von den Verfahrensbedingungen, uneinheitliche Verfahrensprodukte, mangelhafte Reproduzierbarkeit usw.. Die Herstellung dieser Systeme ist verglichen mit konventionellen Mischverfahren technisch aufwendig: So wendet man Hochdruckhomogenisation, Extrusionstechnik, Behandlung mit Ultraschall usw. und entsprechende Gerätetechnik an. In der Regel sind ausserdem nachträgliche Trennverfahren, z.B. Dialysetechnik, Gelfiltration oder Sterilfiltration, erforderlich, bevor man solche Dispersionen applizieren kann.

Es wurde überraschenderweise gefunden, dass das besonders schwerlösliche Staurosporin bzw. seine Derivate mit einfachen konventionellen Mischverfahren feindisperse Systeme in der für intravenöse Darreichungsformen erforderlichen Homogenität und Stabilität bilden können, wenn man ein Polyoxyethylen-polyoxypropylen Blockcopolymer und gegebenenfalls weitere zulässige Hilfsstoffe hinzufügt.

Gegenstand der vorliegenden Erfindung ist eine Formulierungsgrundlage für die Herstellung einer pharmazeutischen Zusammensetzung für die intravenöse Applikation von Staurosporin-Derivaten enthaltend:
a) ein in Wasser schwerlösliches Staurosporin-Derivat;
b) ein Polyoxyethylen-polyoxypropylen-Blockcopolymer;
c) Ethanol und Wasser als Trägerflüssigkeiten in der für die intravenöse Applikation erforderlichen Reinheit; und gegebenenfalls
d) ein Phospholipid der Formel 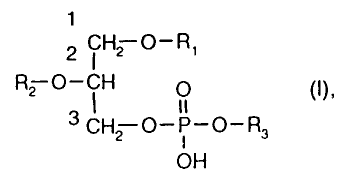 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅- Alkyl substituiert durch Carboxy, C₂₋₅-Alkyl substituiert durch Hydroxy, C₂₋₅-Alkyl substituiert durch Carboxy und Hydroxy, C₂₋₅-Alkyl substituiert durch Carboxy und Amino, oder die Inositol- oder die Glycerylgruppe bedeuten, oder Salze dieser Verbindungen; und/oder
e) für Injektabilia geeignete wasserlösliche Hilfsstoffe.

Die weiter vorn definierte pharmazeutische Zusammensetzung zeichnet sich durch günstige Phaseneigenschaften des solubilisierten Wirkstoffs aus. So ist bei vorhandener Opaleszenz und Transparenz im Gegenlicht nur an einer äusserst geringen milchigen Trübung zu erkennen, dass die gebildete Dispersion noch physikalische Unterschiede gegenüber dem Idealzustand einer echten molekularen Lösung aufweist. Elektronenmikroskopische Abbildungen zeigen, dass eine Population von mehr als 95% des schwerlöslichen Wirkstoffs als Suspension von Partikeln mit einer Teilchengrösse von ca. 5-20 nm ("Nanosuspension") vorliegt. Diese Unterschiede gegenüber einer echten Lösung sind aber aufgrund der besonders guten Homogenitätseigenschaften der Dispersion hinnehmbar, die beispielsweise an einer überraschend hohen Lagerstabilität, z.B. keine Entmischung nach mehrmonatiger Lagerung bei 2-8°C (durch Extrapolation zu erwartende Stabilität länger als zwei Jahre) nachweisbar sind.

Eine besonders bevorzugte Ausführungsform betrifft eine Formulierungsgrundlage für die Herstellung einer pharmazeutischen Zusammensetzung enthaltend:
a) den Wirkstoff N-Benzoyl-Staurosporin;
b) ein Polyoxyethylen-polyoxypropylen-Blockcopolymer;
c) Ethanol und Wasser als Trägerflüssigkeiten; und
d) gereinigtes Lecithin aus Sojabohnen und
e) als wasserlösliche Hilfsstoffe Glycerin und Sorbitol.

Eine weitere bevorzugte Ausführungsform betrifft eine Formulierungsgrundlage zur Herstellung einer pharmazeutischen Zusammensetzung enthaltend:
a) den Wirkstoff N-Benzoyl-Staurosporin;
b) ein Polyoxyethylen-polyoxypropylen-Blockcopolymer;
c) Ethanol und Wasser als Trägerflüssigkeiten; und
e) als wasserlöslichen Hilfsstoff Glycerin.

Eine ganz besonders bevorzugte Ausführungsform betrifft eine Formulierungsgrundlage zur Herstellung einer pharmazeutischen Zusammensetzung enthaltend:
a) den Wirkstoff N-Benzoyl-Staurosporin;
b) Poloxamer 188;
c) Ethanol und Wasser als Trägerflüssigkeiten;
d) gereinigtes Lecithin aus Sojabohnen und
e) als wasserlösliche Hilfsstoffe Glycerin und Sorbitol.

Eine weitere ganz besonders bevorzugte Ausführungsform betrifft eine Formulierungsgrundlage zur Herstellung einer pharmazeutischen Zusammensetzung enthaltend:
a) den Wirkstoff N-Benzoyl-Staurosporin;
b) Poloxamer 188;
c) Ethanol und Wasser als Trägerflüssigkeiten; und
e) als wasserlöslichen Hilfsstoff Glycerin.

### Komponente a)

Ein in Wasser schwerlösliches Staurosporinderivat ist beispielsweise in der U.S. Patentschrift 5 093 330 beschrieben und leitet sich vom Staurosporin durch zusätzliche Substitution des freien Wasserstoffatoms am Stickstoff des N-Methylamino-Substituenten ab. Für Staurosporinderivate ist die schlechte Löslichkeit in Wasser charakteristisch, was sie für intravenöse Darreichungsformen ungeeignet erscheinen lässt. So besitzt das besonders wirksame N-Benzoylstaurosporin bei Raumtemperatur die geringe Wasserlöslichkeit von weniger als 0,1mg/l.

Geeignete Staurosporin-Derivate sind beispielsweise N-(3-Nitrobenzoyl)-staurosporin, N-(3-Fluorbenzoyl)-staurosporin, N-Trifluoracetyl-staurosporin, N-Phenylcarbamoyl-staurosporin, N-(3-Carboxypropionyl)-staurosporin, N-Methylaminothiocarbonyl-staurosporin, N-tert--Butoxycarbonyl-staurosporin, N-(4-Carboxybenzoyl)-staurosporin, N-(3,5-Dinitrobenzoyl)-staurosporin, N-(2-Aminoacetyl)-staurosporin, N-Alanylstaurosporin sowie pharmazeutisch annehmbare Salze dieser Derivate. Besonders bevorzugt ist das N-Benzoylstaurosporin-Derivat.

### Komponente b)

Das Polyoxyethylen-polyoxypropylen-Blockcopolymer wird auch als Poloxamer bezeichnet, siehe entsprechendes Stichwort in Hagers Handbuch der Pharmazeutischen Praxis, 5. Auflage. Es ist ein Blockcopolymeres aus Ethylenoxid und Propylenoxid, das kommerziell erhältlich ist und Molekülmassen von ca. 100 bis 16 000 aufweist. Darin beträgt der Polymerisationsgrad der Ethylenoxideinheiten ca. 10 - 110 und der Propylenoxideinheiten ca. 20 - 60. Geeignet sind hauptsächlich die in USP XXII mit den Bezeichnungen Poloxamer 124,188, 237, 338 und 407 versehenen Typen.

Ein besonders bevorzugtes Polyoxyethylen-polyoxypropylen-Blockcopolymeres ist unter der Bezeichnung Poloxamer 188 bekannt und kommerziell (BASF) unter den Bezeichnungen Pluronic® F68 und Lutrol® F68 erhältlich.

### Komponente c)

Die Trägerflüssigkeit Ethanol ist in der gemäss Vorschrift der nationalen Pharmakopöen, wie U.S. Pharmakopöe (USP) oder Deutsches Arzneibuch (DAB), für Injektionsformulierungen vorgeschriebenen Reinheit (96 %) enthalten. Der Mengenanteil Ethanol kann in weiten Bereichen von ca. 1 % bis ca 50 %, vorzugsweise von ca. 1 % bis ca.10 %, variieren. Die zweite Trägerflüssigkeit, Wasser, ist in der für die intravenöse Applikation erforderlichen Reinheit nach den Vorschriften der nationalen Pharmakopöen keim- und pyrogenfrei.

### Komponente d)

Die Nomenklatur der Phospholipide (1) und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

R₁ und R₂ mit den Bedeutungen C₁₀₋₂₀-Acyl sind vorzugsweise geradkettiges C₁₀₋₂₀-Alkanoyl mit einer geraden Anzahl an C-Atomen und geradkettiges C₁₀₋₂₀-Alkenoyl mit ein bis drei Doppelbindungen und einer geraden Anzahl an C-Atomen.

Geradkettiges C₁₀₋₂₀-Alkanoyl R₁ und R₂ mit einer geraden Anzahl an C-Atomen sind beispielsweise n-Dodecanoyl, n-Tetradecanoyl, n-Hexadecanoyl oder n-Octadecanoyl.

Geradkettiges C₁₀₋₂₀-Alkenoyl R₁ und R₂ mit ein bis drei Doppelbindungen und einer geraden Anzahl an C-Atomen sind beispielsweise 6-cis-, 6-trans-, 9-cis- oder 9-trans-Dodecenoyl, -Tetradecenoyl, -Hexadecenoyl, -Octadecenoyl oder -Icosenoyl, insbesondere 9-cis-Octadecenoyl (Oleoyl), ferner 9,12-cis-Octadecadienoyl oder 9,12,15-cis-Octadecatrienoyl.

Ein Phospholipid (I), worin R₃ 2-Trimethylamino-1-ethyl bedeutet, wird mit dem Trivialnamen Lecithin und ein Phospholipid (I), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen R₁ und R₂, oder Mischungen davon.

Das Phospholipid (I) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter vom definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (= Oleoyl).

Der Begriff "natürlich vorkommendes" Phospholipid (1) definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ strukturell undefinierbar und von natürlich vorkommenden Fettsäuregemischen abgeleitet sind.

Die Forderung "im wesentlichen reines" Phospholipid (I) definiert einen Reinheitsgrad von mehr als 90% (Gew.), vorzugsweise mehr als 95%, des Phospholipids (I), welcher anhand geeigneter Bestimmungsmethoden, z.B. papierchromatographisch, mit Dünnschichtchromatographie, mit HPLC oder enzymatischem Farbtest, nachweisbar ist.

In einem Phospholipid (I) ist R₃ mit der Bedeutung C₁₋₄-Alkyl beispielsweise Methyl oder Ethyl. Die Bedeutung Methyl ist bevorzugt.

R₃ mit den Bedeutungen C₁₋₅-Alkyl substituiert durch Carboxy, C₂₋₅-Alkyl substituiert durch Hydroxy oder C₂₋₅-Alkyl substituiert durch Carboxy und Hydroxy sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl.

R₃ mit der Bedeutung C₂₋₅-Alkyl substituiert durch Carboxy und Amino ist z.B. 3-Amino-3-carboxy-n-propyl oder 2-Amino-2-carboxy-n-propyl, vorzugsweise 2-Amino- 2-carboxyethyl.

Ein Phospholipid (I) mit diesen Gruppen kann in Salzform, z.B. als Natrium- oder Kaliumsalz, vorliegen.

Phospholipide (I), worin R₃ die Inositol- oder die Glycerylgruppe bedeutet, sind unter den Bezeichnungen Phosphatidylinositol und Phosphatidylglycerol bekannt.

Für die Acylreste in den Phospholipiden (I) sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 11-cis-Octadecenoyl (Vaccenoyl), 9-cis-lcosenoyl (Gadoleoyl), n-Dodecanoyl (Lauroyl), n-Tetradecanoyl (Myristoyl), n-Hexadecanoyl (Palmitoyl), n-Octadecanoyl (Stearoyl), n-lcosanoyl (Arachidoyl), n-Docosanoyl (Behenoyl), n-Tetracosanoyl (Lignoceroyl).

Ein Salz des Phospholipids (1) ist pharmazeutisch annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere das Natriumsalz.

In einer besonders bevorzugten Ausführungsform verwendet man gereinigtes Lecithin aus Sojabohnen, z.B. vom Typ LIPOID S 100

### Komponente e)

Für Injektabilia geeignete wasserlösliche Hilfsstoffe sind in der pharmazeutischen Zusammensetzung und der Formulierungsgrundlage wahlweise vorhanden. Besonders bevorzugt ist wasserfreies Glycerin. Es können ausserdem noch Hilfsstoffe zur Herstellung von isotonischen Bedingungen, z.B. ionische Hilfsstoffe, z.B. Natriumchlorid, oder andere wasserlösliche Hilfsstoffe vom Typ pharmazeutisch annehmbare Hexose, z.B. Sorbitol, Mannit, Glucose, Lactose oder Sorbitan vorhanden sein.

In einer bevorzugten Ausführungsform ist wasserfreies Glycerin und zusätzlich Sorbitol enthalten.

Ebenfalls Gegenstand der Erfindung ist die Weiterverarbeitung der Formulierungsgrundlage zur Herstellung einer pharmazeutischen Zusammensetzung und das Verfahren zur Herstellung der Formulierungsgrundlage, welche dadurch gekennzeichnet ist, dass man eine wässrige Dispersion durch homogenes Vermischen der Komponenten b) und c) und gegebenenfalls d) und/oder e) herstellt und die erhältliche Dispersion folgenden Nachoperationen unterzieht: α) Zusatz einer weiteren Menge Wasser als Trägerflüssigkeit sowie gegebenenfalls weiterer wasserlöslicher Hilfsstoffe, welche für Injektabilia geeignet sind, Filtration und gegebenenfalls Dialyse der klaren Dispersion; oder β) Filtration und gegebenenfalls Dialyse und anschliessende Überführung der erhältlichen Dispersion in ein Trockenpräparat, gegebenenfalls unter Zusatz von wasserlöslichen Hilfsstoffen, und Rekonstitution des Trockenpräparates.

In einer besonders bevorzugten Ausführungsform des Verfahrens stellt man eine intravenös applizierbare Dispersion mit Nanopartikeln des in Wasser schwerlöslichen Staurosporinderivates N-Benzoyl-staurosporin her.

In einer ebenfalls besonders bevorzugten Ausführungsform des Verfahrens verwendet man als Formulierungsgrundlage das Polyoxyethylen-polyoxypropylen-Blockcopolymer der Komponente b), kombiniert mit einem Phospolipid der Formel 1 (Komponente d)), und setzt zu den Trägerflüssigkeiten Ethanol und Wasser (Komponente c)) Glycerin und eine pharmazeutisch annehmbare Hexose, z.B. Sorbitol, als wasserlösliche Hilfsstoffe der Komponente e) hinzu.

Gegenstand der vorliegenden Erfindung ist die für die bevorzugte Ausführungsform des Verfahrens verwendbare Formulierungsgrundlage enthaltend:
b) ein Polyoxyethylen-polyoxypropylen-Blockcopolymer;
c) Ethanol und Wasser als Trägerflüssigkeiten; sowie
d) ein Phospholipid der Formel 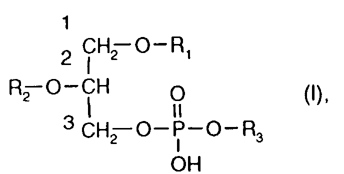 worin R₁, R₂ und R₃ die genannten Bedeutungen haben; und gegebenenfalls
e) als wasserlösliche Hilfsstoffe Glycerin und/oder eine pharmazeutisch annehmbare Hexose;

Diese Formulierungsgrundlage ist sowohl für intravenöse als auch für solche Darreichungsformen geeignet, worin die Solubilisierung eines schwerlöslichen Wirkstoffs erforderlich ist, z.B. Kapselfüllungen, Tropfen, Lotionen oder Emulsionen für Salben, Gele, Crèmes etc.. Letzteren kann man noch die für diese Darreichungsformen charakteristischen weiteren Hilfsstoffe beifügen. Die Formulierungsgrundlage ist sowohl für die Solubilisierung von schwerlöslichen Staurosporin-Derivaten im Sinne der beschriebenen Aufgabenstellung als auch für die Solubilisierung von anderen schwerlöslichen Wirkstoffen verwendbar.

Besonders bevorzugt ist die Formulierungsgrundlage enthaltend
b) ein Polyoxyethylen-polyoxypropylen-Blockcopolymer;
c) Ethanol und Wasser als Trägerflüssigkeiten; sowie
d) gereinigtes Lecithin aus Sojabohnen und gegebenenfalls
e) als wasserlösliche Hilfsstoffe Glycerin und/oder Sorbitol.

Gemäss einer besonders bevorzugten Verfahrensvariante stellt man eine intravenös applizierbare Dispersion mit Nanopartikeln des in Wasser schwerlöslichen Staurosporinderivates N-Benzoyl-staurosporin und folgender Formulierungsgrundlage her:
b) Poloxamer 188;
c) Ethanol und Wasser als Trägerflüssigkeiten;
d) gereinigtes Lecithin aus Sojabohnen und
e) als wasserlösliche Hilfsstoffe Glycerin und Sorbitol.

Bei der Herstellung dieser Dispersion legt man das Lecithin aus Sojabohnen in Ethanol vor, fügt langsam den Wirkstoff N-Benzoyl-staurosporin und danach Glycerin und eine konzentrierte wässrige Lösung von Sorbitol hinzu. Man füllt mit Wasser (aqua ad inj.) auf das für Injektions- oder Infusionslösungen erforderliche Volumen auf.

Das Vermischen kann durch intensives Schütteln mit einem Dispergator, z.B. einem Mischer der Fa. Vortex, oder Dispergatoren vom Typ POLYTRON (Kinematica AG, Littau CH) oder Dispergatoren der Fa. IKA (DE-Staufen), einem statischen Mischer sowie konventionellen Rührern mit Propeller oder Flügelblatt oder einem Magnetrührer oder Phasenmischer erfolgen. Um eine besonders homogene Mischung zu erhalten, rührt man hochtourig, z.B. mit Rührern der Fa. Polytron, z.B. Polytron PT 3000 oder DH 30/30. Es können ca. 0,1 bis 50 Gewichtsprozent der Bestandteile ohne die Komponente Wassser, bezogen auf das Gesamtgewicht der Suspension, vorzugsweise ca. 2 bis 20 Gewichtsprozent, in wässriger Phase dispergiert werden. Bei der Verwendung von Phospholipiden (Komponente d)) ist die sogenannte Phasenübergangstemperatur (gelförmig/flüssigkdstallin) der verwendeten Phospholipide kritisch. Man dispergiert vorzugsweise bei Temperaturen, worin das verwendete Phospholipid in flüssigkristallinem Zustand vorliegt, also oberhalb der sogenannten Phasenübergangstemperatur. Besonders geeignet ist ein Phospholipid, das bei Raumtemperatur oder niedrigeren Temperaturen in flüssigkristallinem Zustand vorliegt, z. B. Lecithin aus Sojabohnen.

Die erhältliche Mischung lässt sich als Suspension von kolloiden Nanopartikeln oder vereinfacht als Nanosuspension definieren. Aufgrund von Laser-Streulichtmessungen und Aufnahmen im Elektronenmikroskop sind die in der Suspension vorhandenen kolloiden Partikel von anderen Gebilden wie Flüssigkristallen, Mizellen, Umkehrmizellen oder Liposomen unterscheidbar. Für die statistische Mehrzahl von mehr als 90%, vorzugsweise mehr als 95%, ist eine mittlere Partikelgrösse kleiner als 20 nm charakteristisch.

Zur Charakterisierung der erhältlichen Nanosuspensionen sind an sich bekannte Methoden geeignet, z.B. durch optische Beurteilung. Eine schwache bis starke Opaleszenz des Präparats ist leicht erkennbar (Hinweis auf durchschnittliche Partikelgrösse kleiner als 20 nm); Laser-Lichtstreuung (Bestimmung der Partikelgrösse und Homogenität); Elektronenmiskroskopie (Gefrierbruch- und Negativkontrasttechnik).

### Nachoperationen)

Der Nanosuspension kann man die gewünschte Menge Wasser, welches in der für Injektionen geforderten Reinheit vorliegen muss, hinzusetzen, so dass die Nanosuspension nach Wahl einer für solche Dispersionen geeigneten Filtrationsmethode, z.B. steriler Gelfiltration, z.B. mit Sepharose® oder Sephacryl® (Pharmacia) als Träger, oder vorzugsweise Sterilfiltration (0,2 µm), z.B. mit PAL-Filter (Gelman) und gegebenenfalls nach Zusatz von weiteren wasserlöslichen Hilfsstoffen, die für intravenöse Darreichungsformen verwendbar sind, direkt applizierbar ist. Besonders durch Sterilfiltration kann man alle in der Dispersion befindlichen grösseren Partikel mit einem Durchmesser grösser als ca. 200 nm, sowie Schweb- und Feststoffe, überschüssige, dispergierte Lipide, welche in hochmolekularen Aggregaten vorliegen können, abtrennen und so eine Nanosuspension mit einer Fraktion von hydrophilen Partikeln mit relativ einheitlicher Grösse herstellen. Altemativ oder zusätzlich zur Sterilfiltration kann man die Nanosuspension zwecks Reinigung dialysieren und/oder ultrafiltrieren.

Alternativ zur Herstellung einer direkt applizierbaren Nanosuspension kann man die weiter vom beschriebenen nachträglichen Reinigungsoperationen durchführen und die gereinigte Nanosuspension in ein Trockenpräparat, insbesondere in ein Lyophilisat, überführen, welches man vor der Applikation durch Zusatz von Wasser rekonstituiert. Auch nach Rekonstitution des Lyophilisats erhält man wieder eine applizierbare Nanosuspension. Für die Herstellung von Lyophilisaten ist der Zusatz von sogenannten Gerüstbildnern, wie Lactose oder Mannit, üblich. Dieser Hilfsstoff wird in geeigneter Menge hinzugefügt, so dass nach Rekonstitution des Lyophilisats die zu applizierende Nanosuspension isotonische Bedingungen aufweist.

Abgemessene Mengen Nanosuspension füllt man, gegebenenfalls als Konzentrat, in geeignete Behälter für eine Dosiseinheit, z.B. Glasstechampullen (Vials), ab. Die abgefüllten Behälter kann man gewünschtenfalls auf ca. -40° bis -50°C, insbesondere auf ca.-45°C, abkühlen und anschliessend bei einem Druck von ca. 0,2 bis 0,6 mbar durch langsames Erwärmen bis zu einer Endtemperatur von ca. 25° bis 35°C lyophilisieren.

Die weiter vom beschriebenen pharmazeutischen Zusammensetzungen sind als intravenös verabreichbare Arzneimittel zur Behandlung von Krankheiten, die von malignem Zellwachstum verursacht werden, verwendbar. Insbesondere eignen sie sich als Tumorhemmer, als Antiinflammatorika, als Antibiotika, bei der Behandlung von Arteriosklerose oder sind bei diversen Erkrankungen des kardiovaskulären Systems und des zentralen Nervensystems therapeutisch anwendbar.

Die folgenden Beispiele illustrieren die Erfindung:

### Beispiel 1: Rezeptur für 20 Injektionsformulierungen à 5 ml und 100 mg Wirkstoff

| | |
|---|---|
| 2,0 g | N-Benzoyl-staurosporin |
| 10,0 g | LUTROL F68 |
| 2,0 g | Lecithin aus Sojabohnenöl (LIPOID S 100) |
| 30,0 g | Glycerin (wasserfrei) |
| 21,0 g | Sorbitollösung 70% (w/w) |
| 35,0 g | Ethanol (abs. 96%-ig) |
| **100,0 g** | **Ansatz** |

Man legt LIPOID S 100 vor, löst in Ethanol und rührt bei Raumtemperatur mit einem Magnetrührer. Hierzu gibt man LUTROL F68 und rührt bei ca. 35°C. Man gibt zu diesem Ansatz den Wirkstoff N-Benzoyl-staurosporin und rührt 10 Min. lang ebenfalls bei 35°C. Anschliessend vermischt man mit dem Glycerin und rührt bei Raumtemperatur, bis die Mischung klar wird. Danach fügt man die 70%-ige Sorbitollösung hinzu, welche man zuvor durch Auflösung von Sorbitol in Wasser hergestellt hat. Das Gemisch wird wieder mit dem Magnetrührer gemischt, bis die Mischung klar wird. Diese wird anschliessend keimfiltriert (Porenfilter: 0,2 µm) und steril abgefüllt. Die Lagerung erfolgt anschliessend bei 4-7°C.

### Beispiel 2: Herstellung einer Infusionslösung

Der Ansatz gemäss Beispiel 1 lässt sich auch zur Herstellung von Infusionslösungen à 250 ml verwenden: Man legt 235 g 5%-ige Glucose-Lösung oder 0,9%-ige NaCl-Lösung bei Raumtemperatur vor und setzt 15 ml der gemäss Beispiel 1 hergestellten Lösung hinzu. Die Infusionslösung wird anschliessend keimfiltriert (Porenfilter: 0,2 µm) und steril abgefüllt. Die Infusionslösung hat folgende Konzentrationen:

| | |
|---|---|
| 0,12 % | N-Benzoyl-staurosporin |
| 0,60 % | LUTROL F68 |
| 0,12 % | Lecithin aus Sojabohnenöl (LIPOID S 100) |
| 1,80 % | Glycerin (wasserfrei) |
| 0,88 % | Sorbitollösung 70% (w/w) |
| 2,10 % | Ethanol (abs. 96%-ig) |

### Beispiel 3: Herstellung einer Infusionsformulierung

Man stellt eine Lösung bestehend aus 600 mg LUTROL F68, 1200 mg Ethanol und 2500 mg Glycerin her und versetzt diese mit 1500 mg N-Benzoyl-staurosporin. Diese Mischung wird mit einer Pumpgeschwindigkeit von 0,5 - 7,5 ml/min. einem statischen Mischer (drei Elemente SMX mit Durchmesser 3,2 mm) zugeführt. Anschliessend vermischt man mit 95,55 g einer wässrigen Lösung enthaltend 0,9 % NaCl oder 5 % Glucose, Sorbitol oder Mannitol, welche man mit einer Pumpgeschwindigkeit von 50 bis 75 ml/Min. hinzufügt. Die Infusionslösung hat pro ml folgende Bestandteile:

| | |
|---|---|
| 1,5 mg | N-Benzoyl-staurosporin |
| 6,0 mg | LUTROL F68 |
| 12,0 mg | Ethanol |
| 25,0 mg | Glycerin |

## Patentansprüche

1. Formulierungsgrundlage enthaltend
b) ein Polyoxyethylen-polyoxypropylen-Blockcopolymer;
c) Ethanol und Wasser als Trägerflüssigkeiten ; sowie
d) ein Phospholipid der Formel 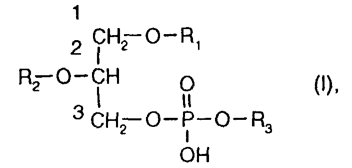 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅- Alkyl substituiert durch Carboxy, C₂₋₅-Alkyl substituiert durch Hydroxy, C₂₋₅-Alkyl substituiert durch Carboxy und Hydroxy, C₂₋₅-Alkyl substituiert durch Carboxy und Amino bedeuten, oder die Inositol- oder Glycerylgruppe oder Salze dieser Verbindungen; und/oder
e) als wasserlösliche Hilfsstoffe Glycerin und eine pharmazeutisch annehmbare Hexose.

2. Formulierungsgrundlage gemäss Anspruch 1 enthaltend:
b) ein Polyoxyethylen-polyoxypropylen-Blockcopolymer;
c) Ethanol und Wasser als Trägerflüssigkeiten;
d) gereinigtes Lecithin aus Sojabohnen und/oder;
e) als wasserlösliche Hilfsstoffe Glycerin und Sorbitol.

3. Verfahren zur Herstellung einer pharmazeutischen Formulierungsgrundlage gemäss Anspruch 1, dadurch gekennzeichnet, dass man eine wässrige Dispersion durch homogenes Vermischen der Komponenten b) und c) und gegebenenfalls d) und/oder e) herstellt und die erhältliche Dispersion folgenden Nachoperationen unterzieht: α) Zusatz einer weiteren Menge Wasser als Trägerflüssigkeit sowie gegebenenfalls weiterer wasserlöslicher Hilfsstoffe, welche für Injektabilia geeignet sind, Filtration und gegebenenfalls Dialyse der klaren Dispersion; oder β) Filtration und gegebenenfalls Dialyse und anschliessende Überführung der erhältlichen Dispersion in ein Trockenpräparat, gegebenenfalls unter Zusatz von wasserlöslichen Hilfsstoffen, und Rekonstitution des Trockenpräparates.

4. Verfahren gemäss Anspruch 3, dadurch gekennzeichnet, dass man die Formulierungsgrundlage zu einer eine intravenös applizierbare Dispersion mit Nanopartikeln des in Wasser schwerlöslichen Staurosporinderivates N-Benzoyl-staurosporin weiterverarbeitet.
